(19) Europäisches Patentamt / European Patent Office / Office européen des brevets

(11) **EP 4 775 032 A1**

(12) **EUROPEAN PATENT APPLICATION**
published in accordance with Art. 153(4) EPC

(43) Date of publication:
**15.07.2026 Bulletin 2026/29**

(21) Application number: **23951411.0**

(22) Date of filing: **04.09.2023**

(51) International Patent Classification (IPC):
**A01M 29/30** (2011.01)    **A01M 29/10** (2011.01)
**E02B 1/00** (2006.01)

(52) Cooperative Patent Classification (CPC):
**A01M 29/10; A01M 29/30; C02F 1/00; C02F 1/32;
E02B 1/00; G01N 33/18**

(86) International application number:
**PCT/JP2023/032193**

(87) International publication number:
**WO 2025/052506 (13.03.2025 Gazette 2025/11)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
- **The Chugoku Electric Power Co., Inc.**
  **Hiroshima-shi, Hiroshima 730-8701 (JP)**
- **Sessile Research Corporation**
  **Himeji-shi, Hyogo 672-8023 (JP)**

(72) Inventors:
- **YANAGAWA, Toshiharu**
  **Hiroshima-shi, Hiroshima 730-8701 (JP)**
- **NISHIDA, Yurika**
  **Hiroshima-shi, Hiroshima 730-8701 (JP)**

- **YAMASHITA, Keiji**
  **Himeji-shi, Hyogo 672-8023 (JP)**
- **KAMIYA, Kyoko**
  **Himeji-shi, Hyogo 672-8023 (JP)**
- **OTA, Maki**
  **Himeji-shi, Hyogo 672-8023 (JP)**
- **SAITO, Shinsuke**
  **Himeji-shi, Hyogo 672-8023 (JP)**
- **FUKUMOTO, Kazumi**
  **Himeji-shi, Hyogo 672-8023 (JP)**
- **HAYASHI, Yoshio**
  **Himeji-shi, Hyogo 672-8023 (JP)**
- **FUKASAWA, Yuuki**
  **Himeji-shi, Hyogo 672-8023 (JP)**

(74) Representative: **HGF**
**HGF Europe LLP
Neumarkter Straße 18
81673 München (DE)**

(54) **ATTACHING AND PROPAGATION PREVENTION METHOD, ATTACHING AND PROPAGATION PREVENTION DEVICE, AND WATER QUALITY METER**

(57)    Provided are: an attaching and propagation prevention method and an attaching and propagation prevention device capable of efficiently preventing attaching organisms from attaching to and/or propagating on an object, and prolonging the life of a radiation device; and a water quality meter. The attaching and propagation prevention method is for preventing attaching organisms from attaching to and/or propagating on an object which the attaching organisms are capable of attaching to. Said method satisfies the condition represented by expression (1), and in said method, the object is intermittently irradiated with light. Expression (1): $X \times a/(a+b) \geq Y$, where X is the irradiance ($Wm^{-2}$) of the light, a is the time period in which the light is radiated, b is the time period in which the light is not radiated, and Y is the irradiance ($Wm^{-2}$) set per each attaching organism.

FIG. 6

**Description**

TECHNICAL FIELD

The present invention relates to an attaching and propagation prevention method, an attaching and propagation prevention device, and a water quality meter.

BACKGROUND ART

In recent years, technology has been known for preventing the attachment of sessile organisms such as barnacles to structures such as water quality meters, or the seawater system equipment of thermal power plants, nuclear power plants, etc. For example, a method has been known in Patent Document 1 that prevents the attachment of sessile organisms to structures, by irradiating light including a wavelength of 409 to 412 nm onto structures in water.

Citation List

Patent Documents

Patent Document 1: Japanese Unexamined Patent Application, Publication No. 2016-188570

DISCLOSURE OF THE INVENTION

Problems to be Solved by the Invention

The method disclosed in Patent Document 1 can suppress the attachment of sessile organisms to locations where light of high irradiance is irradiated onto a structure. On the other hand, there is a possibility of attracting sessile organisms to locations where light of low irradiance is irradiated such as between light irradiating devices and the ends thereof, and locations where light is not incident and are in the shade, and thus there has been a problem in that the effect of suppressing attachment of sessile organisms is insufficient. In addition, there is a tendency for the irradiation device to deteriorate due to heat generation caused by light irradiation, and thus there is room for improvement in the point of prolonging the lifespan of irradiation devices.
The present invention has been made taking account of the above, and has an object of providing an attaching and propagation prevention method, attaching and propagation prevention device and water quality meter that can effectively suppress attachment of sessile organisms to a target, and realize prolonging of the lifespan of irradiation devices. Means for Solving the Problems

(1) The present invention relates to an attaching and propagation prevention method for suppressing attaching and/or propagation of a sessile organism to a target to which the sessile organism can attach, including: irradiating light onto the target intermittently to satisfy a condition expressed by Formula (1) below.

$$X \times a / (a+b) \geqq Y \ \ldots \ \text{Formula (1)}$$

Herein, X is irradiance of light ($Wm^{-2}$), a is a time for which light is irradiated, b is a time for which light is not irradiated, and Y is an irradiance of light ($Wm^{-2}$) set for every sessile organism.
(2) In the attaching and propagation prevention method as described in (1), Y in the Formula (1) is 20.
(3) In the attaching and propagation prevention method as described in (1), Y in the Formula (1) is 150.
(4) In the attaching and propagation prevention method as described in any one of (1) to (3), light irradiated onto the target has a peak in a wavelength region of 400 to 430 nm.
(5) In addition, the present invention relates to an attaching and propagation prevention device including: an irradiation device that irradiates light onto a target; and a controller that controls the irradiation device, in which the controller controls the irradiation device so that light is intermittently irradiated from the irradiation device onto the target satisfying a condition expressed by Formula (1) below.

$$X \times a / (a+b) \geqq Y \ \ldots \ \text{Formula (1)}$$

Herein, X is irradiance ($Wm^{-2}$) of light from the irradiation device, a is a time for which light is irradiated from the irradiation device, b is a time for which light is not irradiated from the irradiation device, and Y is irradiance of light ($Wm^{-2}$) set for every sessile organism.

(6) Further, the present invention relates to a water quality meter including the attaching and propagation prevention device as described in (5); and a sensor device, in which the irradiation device irradiates light onto a detector of the sensor device, the light irradiated from the irradiation device has a peak in a wavelength region of 400 to 420 nm, the sensor device performs a detection operation at every predetermined detection timing set in advance, and the controller controls the irradiation device so that light is intermittently irradiated onto the detector from the irradiation device every irradiation time outside the predetermined detection timing.

(7) In the water quality meter as described in (6), an irradiation surface of the irradiation device is disposed to oppose the detector.

Effects of the Invention

According to the present invention, it is possible to provide an attaching and propagation prevention method, an attaching and propagation prevention device and a water quality meter that can effectively suppress attachment of sessile organisms to a target, and realize prolonging of the lifespan of irradiation devices.

BRIEF DESCRIPTION OF THE DRAWINGS

FIG. 1 is a block diagram showing the configuration of an attaching and propagation prevention device according to an embodiment of the present invention;
FIG. 2 is a view showing the configuration of a water quality meter according to a first embodiment of the present invention;
FIG. 3 is a view showing the configuration of a water quality meter according to a second embodiment of the present invention;
FIG. 4 is a view showing the configuration of a water quality meter according to a third embodiment of the present invention;
FIG. 5 is a view showing the configuration of a water quality meter according to a fourth embodiment of the present invention;
FIG. 6 is a boxplot related to results of juvenile barnacle rate;
FIG. 7 is a chart showing phototactic states of cypris larva under irradiation conditions of continuous irradiation and intermittent irradiation (1/1, 1/2) of 405 nm wavelength and 20 $Wm^{-2}$ irradiance;
FIG. 8 is a chart showing phototactic states of cypris larva under irradiation conditions of continuous irradiation and intermittent irradiation (1/1, 1/2) of 405 nm wavelength and 400 $Wm^{-2}$ irradiance;
FIG. 9 is a chart showing phototactic states of cypris larva under irradiation conditions of continuous irradiation and intermittent irradiation (1/1, 1/2) of 405 nm wavelength and 600 $Wm^{-2}$ irradiance;
FIG. 10 is a chart showing phototactic states of cypris larva under irradiation conditions of continuous irradiation and intermittent irradiation (1/1, 1/2) of 405 nm wavelength and 2000 $Wm^{-2}$ irradiance;
FIG. 11 is a chart showing phototactic states of cypris larva under irradiation conditions of continuous irradiation and intermittent irradiation (0.1/0.1, 0.1/0.2, 0.1/0.3) of 405 nm wavelength and 600 $Wm^{-2}$ irradiance;
FIG. 12 is a chart showing phototactic states of cypris larva under irradiation conditions of continuous irradiation and intermittent irradiation (1/1, 1/2) of 520 nm wavelength and 600 $Wm^{-2}$ irradiance;
FIG. 13 is a chart showing the reaction situations of freshwater hydra under the respective intermittent irradiation conditions at 500 $Wm^{-2}$ irradiance;
FIG. 14 is a chart showing an evaluation of the reaction situations of freshwater hydra under the respective intermittent irradiation conditions at 1000 $Wm^{-2}$ irradiance;
FIG. 15 is a view for explaining an experimental apparatus used in Example 3 of the present invention;
FIG. 16 is an image showing a test plate after testing related to Comparative Example 6 of the present invention; and
FIG. 17 is an image showing a test plate after testing related to Example 3 of the present invention.

PREFERRED MODE FOR CARRYING OUT THE INVENTION

Hereinafter, embodiments of the present invention will be described. It should be noted that the present invention is not limited to the following embodiments, and modifications are possible where appropriate.

<Attaching and Propagation Prevention Method>

An attaching and propagation prevention method according to the present embodiment is a method of suppressing the attachment of organisms to a target, by intermittently irradiating light (hereinafter called intermittent irradiation) so as to satisfy predetermined conditions on the target on which sessile organism can attach. It should be noted that intermittent irradiation refers to irradiating light by switching between a state in which light is irradiated on the target, and a state not irradiated thereto.

As the target, a structure installed underwater (including structures that are permanently or temporarily wetted by a liquid such as water or sweat) or structures installed in air, for example, seawater system equipment in power generation facilities such as thermal power plants and nuclear power plants, and inspection windows for performing inspection of these facilities can be exemplified. Other than this, the target may be a water quality meter, underwater camera device, water intake pipe, rotary screen, bar screen, drum screen, mussel filter, net screen, water intake pump, circulating water pump, circulating water pipe, heat exchanger, steam condenser, bearing cooling water cooler, lubricating oil cooler, LNG vaporizer, generator, drainage pipe, water turbine, impeller, valve, spindle, water pipeline, filtration tank, membrane, dam, ship, ship hull at shipyard, ballast water tank, ballast water intake/discharge pipes, pumps, aquaculture equipment, fisheries research facility, fishery equipment, aquarium, water tank for seafood breeding tank, plumbing, pumps, cooling tower water tank and radiating plates, scrubber water tank, etc. In addition, for example, it may be the floor or the like of a bathroom, pool, shower room, toilet and kitchen. In addition, it may be indoor seats or in a vehicle, or an operating room of a hospital, or may be a desk or chair, or may be exterior or interior walls of a building, or window glass. In addition, it may be a surface or interior of a pack container, fresh food or artificial food, or may be a foam, cream, jelly, gel or the like. In addition, the structure referenced herein may be a living body. For example, it may be the shell or molluscous part of oysters, fish, protozoa parasitizing fish, plankton, algae, microorganisms, parts of biological tissues, teeth, skin, cells and their secretions. In addition, the structure may or may not be solid. For example, air bubbles in water can be the structure to which many adhesive microorganisms accumulate and adhere to the surface thereof. Similarly, water droplets in air can be the structure in which many adhesive microorganisms accumulate and adhere.

The sessile organism which can adhere to the above-mentioned targets are not particularly limited so long as being an organism having a property of adhering to the surface of a structure in water (including structures that are permanently or temporarily wetted by a liquid such as water or sweat) or a structure in air; however, for example, sessile organism such as mussels, barnacles and hydrozoans, as well as microorganisms such as bacteria, viruses, fungi, blue-green algae and protozoa can be exemplified. The sessile organism is an organism that floats in the sea during the early larval stage, and when becoming sessile larva, attaches to a suitable target and metamorphosizes into an adult. In addition, the larva of microalgae such as attaching diatoms, green algae, brown algae, and red algae, sponges, jellyfish, tubicolous polychaetes, bryozoans, and amphipods are also included therein.

Mussels are a general term for bivalve mollusks of Mytilidae, for example, and includes Modiolinae such as horse mussel, Lithophaginae such as date mussel, Crenellinae such as bag mussel, Mytilinae such as hard-shelled mussel, golden mussel and blue mussel, etc. In addition, barnacle is a general term for things classified as Crustacae, Cirripadia and Thoracica and include, for example, things belonging to Balanomorpha including Amphibalanus amphitrite, American rock barnacle, red barnacle, triangle barnacle, large red barnacle, reticulated barnacle, common sessile barnacle, Mangrove barnacle, and bay barnacle. In addition, hydrozoa is a general term for animals of Cnidaria Hydrozoa and, for example, includes Coryne pusilla, and Anthomedusae Hydridae such as freshwater hydra and green hydra. In addition, as the epiphytic generation of jellyfishes, polyps of moon jellyfish and sea nettles, etc. are included.

In addition, biofilms are also included in the sessile organisms which can attach to the above-mentioned target. Biofilm is a structure formed by microorganisms. Biofilms are usually membrane form, and include extracellular polymeric substances (EPS) such as polysaccharides secreted by microorganisms. In addition, the remains, excretions, feces, dwelling tubes, gene fragments, organic particles, inorganic particles of microorganisms, etc. may be included in biofilms. It should be noted that, in the case of a very thin biofilm being formed on a seat surface or floor surface indoors or in a vehicle, foodstuff, a pack container surface, inner face, internal article, biont, algae, tissue, cells, skin, teeth or the like, this biofilm can be regarded as a sessile organism.

The above predetermined conditions of intermittently irradiating light in the attaching and propagation prevention method are conditions represented by Formula (1) below.

$$X \times a/(a+b) \geqq Y \ \dots \ \text{Formula (1)}$$

However, X is the irradiance of light (Wm$^{-2}$), a is the time for which light is irradiated (hereinafter referred to as lighting time), b is the time for which light is not irradiated (hereinafter referred to as lights-out time), and Y is the irradiance of light (Wm$^{-2}$) set for every sessile organism. In the present disclosure, lighting time indicates a time from when the irradiation of light to the target is started until the irradiation is stopped next in intermittent irradiation. In addition, lights-out time indicates a time from when irradiation stops until irradiation is started next in intermittent irradiation.

As Y, the irradiance capable of preventing the attachment and/or propagation (hereinafter called attaching and propagation prevent) to the target of this sessile organism is set according to the type of sessile organism. The irradiance as Y (hereinafter called irradiance Y) may be set according to the tolerance to light of the sessile organism, for example. In this case, the irradiance Y may be set to the same value for a plurality of sessile organisms having different tolerances to light, for example, or may each be set to different values. In the case of setting to the same value, an irradiance Y effective for attaching and propagation prevention of the sessile organism having the highest tolerance to light may be set. In the present embodiment, the same value is set irrespective of the type of sessile organism. Using the above Formula (1), the irradiation conditions of light for performing attaching and propagation prevention can be set easily.

In the present embodiment, the irradiance Y of Formula (1), for example, is preferably 20 $Wm^{-2}$, and is more preferably 150 $Wm^{-2}$. By adjusting the irradiance X of light, lighting time a and lights-out time b so that the irradiance Y in Formula (1) becomes 20 $Wm^{-2}$, it is possible to prevent attaching and/or propagation of sessile organisms to the target. In addition, by adjusting the irradiance X of light, lighting time a and lights-out time b so that the irradiance Y in Formula (1) becomes 150 $Wm^{-2}$, it is possible to almost completely prevent attaching and/or propagation of sessile organisms to the target. It should be noted that irradiance in the present disclosure shall indicate the value of irradiance measured by an Ophir StarLite PD300Rm-8W radiometer.

The lighting time a and lights-out time b are not particularly limited so long as satisfying the conditions of Formula (1). For example, the lighting time a may be less than 0.1 seconds, may be 0.1 seconds or more, may be 1.0 second or more, may be 1.0 minute or more, or may be 1 hour or more. In addition, for example, the lights-out time b may be less than 0.1 seconds, may be 0.1 seconds or more, may be 1.0 second or more, may be 1.0 minute or more, or may be 1 hour or more. In other words, the lighting time a / (lighting time a + lights-out time b) can be freely set so long as satisfying Formula (1). For example, lighting time a / (lighting time a + lights-out time b) may be set as 0.1, or may be set as 99.9.

The light irradiance X is not particularly limited so long as satisfying the conditions of Formula (1). Irradiance X is preferably 20 $Wm^{-2}$ or more from the viewpoint of preventing attaching and/or propagation of sessile organisms, for example, is more preferably 50 $Wm^{-2}$ or more, and is even more preferably 100 $Wm^{-2}$ or more.

By intermittently irradiating light so as to satisfying the conditions of the aforementioned Formula (1), it is possible to reliably and efficiently prevent the attaching and/or propagation of sessile organisms, and more preferably prevent the attachment of foreign matter to the target of light irradiation, than continuously irradiating light. In detail, in the case of irradiating light continuously, although the foreign matter such as sessile organisms will not attach to locations irradiated with intense light, sessile organisms may be attracted by phototaxis to the peripheral portion of the optical axis on which relatively weak light is irradiated, whereby the sessile organisms propagate. By light being irradiated intermittently onto the target, it is possible to suppress attraction of sessile organisms by phototaxis, and it is possible to efficiently suppress attaching and/or propagation of sessile organisms. In addition, by light being intermittently irradiated onto the target from an irradiation device such as the irradiation device 20 described later, since aging degradation of the LED elements of the irradiation device is suppressed, the durability of the irradiation device can be improved. Consequently, it is possible to achieve prolonged life of the irradiation device.

The light irradiated onto the target preferably has a peak in the wavelength region of 400 to 430 nm. Although ultraviolet rays having a wavelength of 380 nm or less have an attaching and propagation prevention effect for sessile organisms, there is a problem in that the transmittance in seawater is low. By setting the wavelength of irradiated light to the above-mentioned range, the preferred attaching and propagation prevention effect and sessile organism removal effect are obtained. The wavelength of irradiated light may have a peak in a wavelength region outside the above, within a range not inhibiting the effects of the present invention. For example, the wavelength of irradiated light may have a peak in the wavelength region of 430 to 490 nm, or may have a peak in the wavelength region of 490 nm to 550 nm.

Next, examples of a device capable of realizing prevention of attaching or propagation of sessile organisms to a target by the attaching and propagation prevention method will be described.

<Attaching and Propagation Prevention Device>

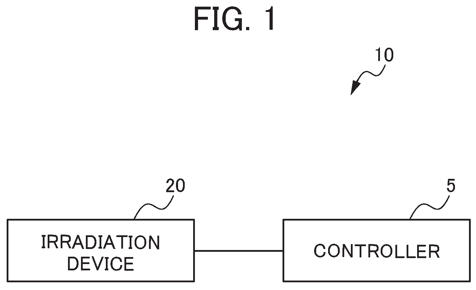

First, an attaching and propagation prevention device 1 will be described while referencing FIG. 1. The attaching and propagation prevent device 1 includes an irradiation device 20 that irradiates light onto the target, and a controller 5 that controls the irradiation device 20, as shown in FIG. 1.

The irradiation device 20 is an LED irradiation device, for example, and is configured by one or a plurality of LED elements. The irradiation device 20 is connected to be able to communicate with the controller 5.

The light irradiated by the irradiation device 20 preferably has a peak in the wavelength region of 400 to 430 nm. The wavelength of irradiated light may have a peak in a wavelength region outside the above, within a range not inhibiting the effects of the present invention. For example, the wavelength of irradiated light may have a peak in the wavelength region of 430 to 490 nm, or may have a peak in the wavelength region of 490 nm to 550 nm.

The controller 5 is an arithmetic unit configured by a processor such as a CPU, and reads out and executes various programs and data established in advance to control the irradiation device 20. The controller 5 suppresses attaching of sessile organisms to the target by controlling the irradiation device 20 so as to satisfy the conditions represented by the above Formula (1), and so that light is intermittently irradiated onto the target from the irradiation device 20. In other words, the irradiation device 20 intermittently irradiates so as to satisfy the conditions of the above Formula (1).

The irradiance X of light irradiated by the irradiation device 20 is not particularly limited so long as satisfying the conditions of Formula (1). The irradiance X is preferably 25 Wm$^{-2}$ or more, more preferably 50 Wm$^{-2}$ or more, and even more preferably 100 Wm$^{-2}$ or more, from the viewpoint of suppressing the attaching of sessile organisms, for example.

<Water Quality Meter>

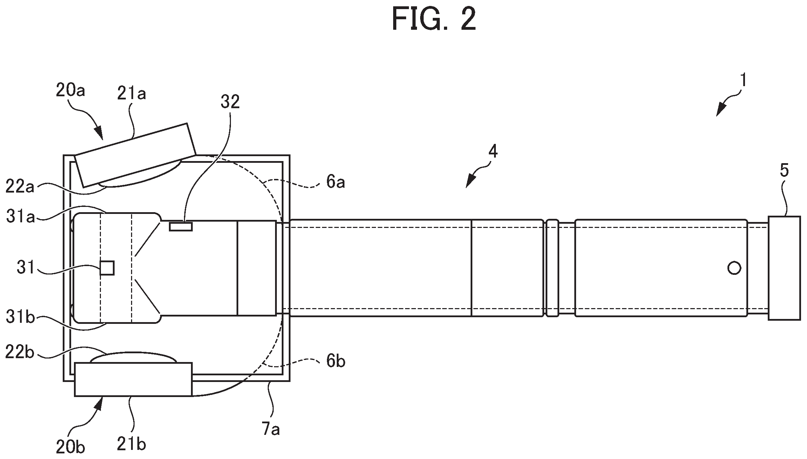

Next, a water quality meter will be described while referencing FIGS. 2 to 5. The water quality meter according to the present embodiment can be used for continuously measuring water in environments such as an ocean, river or lake for a predetermined time period, for example, one day or more. As water quality measurement items measured by the water quality meter, for example, water temperature, salinity, dissolved oxygen (DO) concentration, chlorophyl, organic matter, turbidity, illuminance, pH, ORP (oxidation-reduction potential), conductivity, specific gravity, depth, flow direction and velocity, various ion concentrations, visible light and/or infrared light images, etc. can be exemplified. The water quality meter according to the present embodiment may be capable of measuring a plurality of the above-mentioned water quality measurement items.

The water quality meter according to the present embodiment includes a sensor device as a target, an irradiation device that irradiates light onto a detector of the sensor device, and a controller that controls the irradiation device. It should be noted that a configuration combining the irradiation device and control device of the water quality meter corresponds to the attaching and propagation prevention device 10.

(First Embodiment)

The water quality meter 1 according to the present embodiment is a water temperature salinity meter having a salinity sensor (detector 31) and a water temperature sensor (detector 32) as sensor devices, as shown in FIG. 2. The water quality meter 1, other than the above, includes irradiation devices 20a and 20b, a main body portion 4, a controller 5, and a support 7a.

(Irradiation device)

The irradiation devices 20a and 20b intermittently irradiate light having a peak in the wavelength region of 400 to 430 nm onto the detector 31 of the salinity sensor and the detector 32 of the water temperature sensor. Attaching of sessile organisms to the detector 31 of the salinity sensor and the detector 32 of the water temperature sensor is thereby suppressed. The irradiation devices 20a and 20b are LED irradiation devices, for example, and are each configured by one or a plurality of LED elements. The irradiation devices 20a and 20b respectively include housings 21a and 21b, and irradiation surfaces 22a and 22b. The irradiation surface 22a is arranged to oppose one opening 31a of a hole in which the detector 31 of the salinity sensor is arranged, and the detector 32 of the water temperature sensor. The irradiation surface 22b is arranged to oppose the other opening 31b of the hole in which the detector 31 of the salinity sensor is arranged.

The sessile organisms which can attach to the above-mentioned detectors are not particularly limited so long as being an organism having a property of attaching to the surface of a target arranged underwater; however, for example, mussels, barnacles, hydrozoans and microorganisms such as marine bacteria, biofilms and the like can be exemplified.

The light irradiated from the irradiation devices 20a and 20b has a peak in the wavelength region of 400 to 430 nm. The wavelength of irradiated light may include a wavelength region outside the above within a range not inhibiting the effects of the present invention. For example, it may include light in the wavelength region of 400 to 440 nm.

The irradiance of light irradiated from the irradiation devices 20a and 20b is preferably 25 Wm$^{-2}$ or more, more preferably 50 Wm$^{-2}$ or more, and even more preferably 100 Wm$^{-2}$ or more, from the viewpoint of suppressing the attaching of sessile organisms, for example.

By irradiating light intermittently from the irradiation devices 20a and 20b, it is possible to more favorably suppress attachment of foreign matter onto the detector 31 and the detector 32 irradiated with light, than when continuously irradiating light. In detail, in the case of continuously irradiating light, although foreign matter such as sessile organisms will not attach to locations irradiated by intense light, sessile organisms growing by photosynthesis such as algae will tend to propagate at the peripheral portion of the optical axis on which relatively weak light is irradiated. This is considered to be because the light irradiated from the irradiation devices 20a and 20b includes wavelengths of light effective for photosynthesis. By light being intermittently irradiated from the irradiation devices 20a and 20b, the growth of algae, etc. is suppressed. The reasons for the above are not clear; however, the reason is considered to be that, by light being intermittently irradiated, the light quantity for carrying out photosynthesis of algae, etc. will be insufficient, and thus growth is inhibited.

By light being intermittently irradiated from the above-mentioned irradiation devices 20a and 20b, an effect of suppressing attaching of algae, etc. onto the irradiation surfaces 22a and 22b of the irradiation devices 20a and 20b is obtained. In the case of the irradiation devices 20a and 20b being configured to include a plurality of LED elements, and light being irradiated from the irradiation devices 20a and 20b continuously, the irradiance of irradiated light may decline by algae, etc. attaching between the plurality of LED elements. However, by light being intermittently irradiated from the irradiation devices 20a and 20b, even in the case of the irradiation devices 20a and 20b including a plurality of LED elements, it is possible to suppress the irradiance of irradiated light from declining. In addition, by light being intermittently irradiated from the irradiation devices 20a and 20b, since aging deterioration of the LED elements is suppressed, it is possible to improve the durability of the water quality meter 1.

(Sensor Device)

The salinity sensor is a sensor device that detects the salinity in water. The detector 31 of the salinity sensor is provided in a hole portion through which a liquid can flow. When the flow of liquid such as seawater is inhibited by attachment of sessile organisms to the above-mentioned hole portion, it is not possible to perform accurate measurement of salinity by the salinity sensor. The attachment of sessile organisms to not only the detector 31, but also the hole portion in which the detector 31 is arranged, is suppressed by way of the light irradiated from the irradiation devices 20a and 20b of the water quality meter 1 according to the present embodiment. It is thereby possible to perform continuous and accurate measurement of salinity by the salinity sensor.

The water temperature sensor is a sensor device for detecting water temperature. By the light irradiated from the irradiation device 20a onto the detector 32 of the water temperature sensor, the attachment of sessile organisms to the detector 32 is suppressed. It is thereby possible to continuously and accurately perform measurement of water temperature by the water temperature sensor.

The salinity sensor and water temperature sensor as sensor devices are established so as to perform detection operations every predetermined detection timing that is set in advance. The detection interval at which performing the above-mentioned detection is not particularly limited; however, in order to configure so that the detection results of the sensor device are not influenced by the residual heat from light irradiated from the irradiation device, for example, it is possible to set to an interval of 10 minutes or more. The above-mentioned detection interval may be set to a 1-day interval. The predetermined detection timings of the salinity sensor and water temperature sensor are preferably the same timing.

(Main Body Portion)

The main body portion 4 houses the salinity sensor and water temperature sensor. Other than the above, the main body portion 4 may include a storage portion that stores measurement results from the salinity sensor and the water temperature sensor.

(Controller)

The controller 5 controls the irradiation timings of the irradiation devices 20a and 20b, so that light is intermittently irradiated from the irradiation devices 20a and 20b. The controller 5 is connected to the irradiation devices 20a and 20b to be able to communicate by wiring 6a and 6b, respectively.

The irradiation devices 20a and 20b are controlled by the controller 5, so as to irradiate light onto the detector 31 of the salinity sensor and the detector 32 of the water temperature sensor, every irradiation time other than the predetermined timing at which the above-mentioned salinity sensor and water temperature sensor perform detection operations. In the middle of light being irradiated onto the detector 31 and detector 32 from the irradiation devices 20a and 20b, in the case of the salinity sensor and water temperature sensor performing detection operations, it may not be possible to perform accurate measurement by the salinity sensor and water temperature sensor. However, in the middle of the above-mentioned salinity sensor and water temperature sensor performing detection operations by way of the above configurations, the irradiation devices 20a and 20b are controlled by the controller 5 so as not to irradiate light onto the detector 31 and the detector 32. It is thereby possible to perform accurate measurement by the salinity sensor and water temperature sensor. From the viewpoint of performing accurate measurement, the predetermined timing at which the above-mentioned salinity sensor and water temperatures sensor perform detection operations is preferably set as a timing in the middle of a time period in which light is not irradiated from the irradiation devices 20a and 20b.

The controller 5 preferably controls the irradiation devices 20a and 20b so that a ratio of the irradiation time for which light is irradiated from the irradiation devices 20a and 20b onto the detector 31 and the detector 32 becomes 30 to 70% relative to a total (i.e. elapsed time) of the irradiation time and the time for which light is not irradiated from the irradiation devices 20a and 20b. The effect of preventing attachment of foreign matter such as sessile organisms is thereby obtained, and it is possible to suppress attaching of algae, etc. to locations at which relatively weak light is irradiated.

The controller 5, for example, in the case of the detection interval of the sensor device being a 20-minute interval, and the ratio of the above-mentioned irradiation time being 50%, causes light to be irradiated onto the detector 31 and the detector 32 from the irradiation devices 20a and 20b after 5 minutes elapses from the detection timing of the sensor device. During the irradiation time of 10 minutes, light is continuously irradiated from the irradiation devices 20a and 20b. The controller 5 stops the irradiation of light from the irradiation devices 20a and 20b, when the irradiation time of 10 minutes elapses. Subsequently, the next detection timing of the sensor device is reached after 5 minutes elapses, and the sensor device performs the detection operation. It is thereby possible to eliminate influences on detection of the sensor device such as a water temperature rise due to light being irradiated from the irradiation devices 20a and 20b, for example.

(Support)

The support 7a is a support for fixing the irradiation devices 20a and 20b, and is configured so water from outside can pass relative to the detector 31 and detector 32. The support 7a has a frame shape, for example, is not particularly limited as a material, and a material such as a metal or resin can be employed as appropriate. The irradiation surfaces 22a and 22b can be arranged by the support 7a so as to oppose the detector 31 and the detector 32.

Hereinafter, other embodiments of the present invention will be described. Descriptions may be omitted for configurations which are the same as the above first embodiment.

(Second Embodiment)

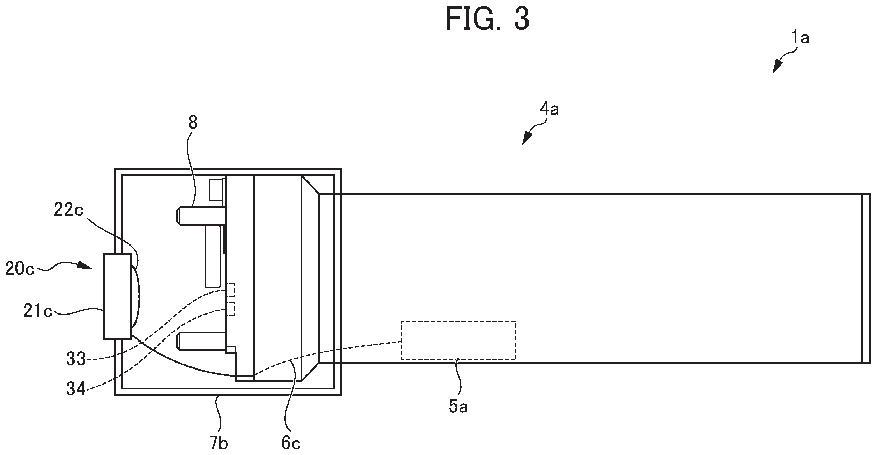

A water quality meter 1a according to a second embodiment includes a dissolved oxygen (DO) concentration sensor (detector 33) and a water temperature sensor (detector 34) as sensor devices, as shown in FIG. 3. The water quality meter 1a, in addition to the above, includes an irradiation device 20c, a main body portion 4a, a controller 5a, a support 7b, and a wiper device 8.

The irradiation device 20c according to the present embodiment includes a housing 21c and an irradiation surface 22c. In the present embodiment, the irradiation device 20c is a single irradiation device, and the irradiation surface 22c is arranged to oppose the detector 33 of the dissolved oxygen (DO) concentration sensor and the detector 34 of the water temperature sensor. The irradiation device 20c is fixed and supported by the support 7b. The support 7b has a frame shape similarly to the support 7a.

The dissolved oxygen (DO) concentration sensor is a sensor device that measures the dissolved oxygen (DO) concentration in water. By light having a peak in the wavelength region of 400 to 430 nm being intermittent irradiated from the irradiation device 20c onto the detector 33 of the dissolved oxygen (DO) concentration sensor and the detector 34 of the water temperature sensor, the attachment of sessile organisms to the above-mentioned detector 33 and detector 34 is suppressed. A configuration in which the detection timings of the dissolved oxygen (DO) concentration sensor and water temperature sensor, and the irradiation timing at which light is irradiated from the irradiation device 20c are controlled by the controller 5a is the same as the water quality meter 1 according to the first embodiment. The controller 5a is housed inside of the main body portion 4a, and is connected to be able to communicate with the irradiation device 20c by wiring 6c.

(Wiper Device)

The wiper device 8 is a device that removes sessile organisms attaching to the detector 33 of the dissolved oxygen (DO) concentration sensor and the detector 34 of the water temperature sensor by wiping off. The wiper device 8 has a contact portion that rotatably abuts the detector 33 and the detector 34. The contact portion is configured from a member having flexibility such as a resin. The water quality meter 1a, by having the wiper device 8 in addition to the irradiation device 20c, even if assuming a case of the attaching and propagation prevention or removal effect of sessile organisms to the detector 33 and detector 34 not being sufficiently obtained by one device due to failure of the device, can perform accurate measurement more reliably with the sensor device, due to the attaching and propagation prevention or removal effect of sessile organisms to the detector 33 and detector 34 being obtained by another device.

(Third Embodiment)

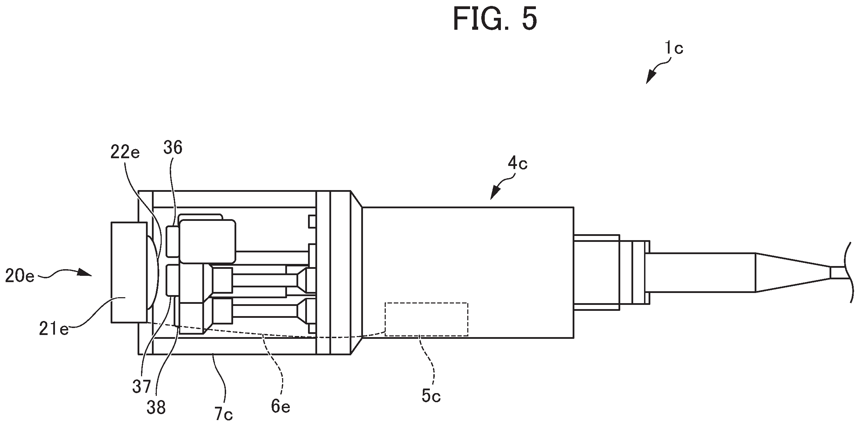

A water quality meter 1c according to a third embodiment includes a pH/ORP sensor (detector 36), a chlorophyll/turbidity sensor (detector 37), and depth sensor (detector 38) as the sensor devices, as shown in FIG. 5. The water quality meter 1c, in addition to the above, includes an irradiation device 20e, a main body portion 4c and a controller 5c.

The irradiation device 20e according to the present embodiment includes a housing 21e and an irradiation surface 22e. In the present embodiment, the irradiation device 20e is a single irradiation device, and the irradiation surface 22e is arranged to oppose the detector 36 of the pH/ORP sensor, the detector 37 of the chlorophyll/turbidity sensor, and the detector 38 of the depth sensor. The irradiation device 20e is fixed and supported by the support 7c. The support 7c has a frame shape similarly to the frame 7a.

The pH/ORP sensor is a sensor device measuring the hydrogen ion exponent in water, and oxidation reduction potential (ORP). The chlorophyll/turbidity sensor is a sensor device measuring the chlorophyll concentration in water and turbidity. The depth sensor is a sensor device measuring the depth from the water surface.

By light having a peak in the wavelength region of 400 to 430 nm being intermittently irradiated from the irradiation device 20e onto the detector 36 of the pH/ORP sensor, the detector 37 of the chlorophyll/turbidity sensor, and the detector 38 of the depth sensor, attachment of sessile organisms to the above-mentioned detector 36, detector 37 and detector 38 is suppressed. A configuration in which the detection timings of the pH/ORP sensor, chlorophyll/turbidity sensor and depth sensor, and the irradiation timing at which light is irradiated from the irradiation device 20e are controlled by the controller 5c is the same as the water quality meter 1 according to the first embodiment. The controller 5c is housed inside of the main body portion 4c, and is connected to be able to communicate with the irradiation device 20e by wiring 6e.

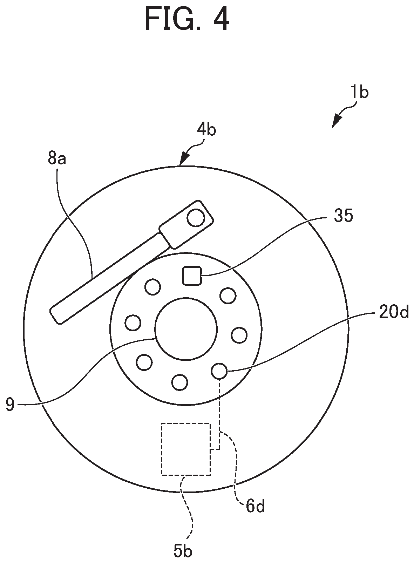

Next, an example applying the attaching and propagation prevention method according to the present embodiment to an underwater camera device will be described. An underwater camera device 1b has an illuminance sensor (detector 35) as a sensor device, as shown in FIG. 4. The underwater camera device 1b, in addition to the above, includes an irradiation device 20d, the main body portion 4b, the controller 5b, the wiper device 8a, and an imaging unit (camera lens 9).

The underwater camera device 1b can regularly capture an underwater image by way of the imaging unit. The above-mentioned underwater image is a visible light image. The underwater camera device 1b includes a plurality of the irradiation devices 20d around the camera lens 9. Light having a peak in the wavelength region of 400 to 430 nm can be irradiated from the irradiation device 20d, and preferably white light can be further irradiated.

The lighting time a and the lights-out time b of light having a peak in the wavelength region of 400 to 430 nm irradiated from the irradiation device 20d are not particularly limited so long as satisfying the condition of Formula (1). In other words, so long as satisfying Formula (1), the controller 5b can freely set the lighting time a / (lighting time a + lights-out time b). For example, the lighting time a / (lighting time a + lights-out time b) may be set as 0.1 or may be set as 99.9.

By irradiating light from the irradiation device 20d according to the imaging timing of the imaging unit, it may be employed as lighting. For example, in the case of having low illuminance around the imaging timing of the imaging unit, a case of irradiating white light from the irradiation device 20d can procure an image including color information of an imaging target by irradiating white light as lighting.

In the case of having low illuminance around the imaging timing of the imaging unit, a case of irradiating light having a peak in the wavelength region of 400 to 430 nm from the irradiation device 20d can capture an underwater image related to a relatively far region, due to the light having a peak in the wavelength region of 400 to 430 nm being light having a wavelength region with high permeability in seawater.

A plurality of the irradiation devices 20d (seven in the present embodiment) is provided around the camera lens 9 in the present embodiment.

The illuminance sensor (detector 35) measures the surrounding illuminance, and turns on illumination from the irradiation device 20d at conditions of lower illuminance (darker) of the surroundings than a predetermined value.

Next, another example applying the attaching and propagation prevention method according to the present embodiment will be shown. In the example applied to the aforementioned underwater camera device, attaching and propagation prevention is performed using the irradiation device 20 by which light is intermittently irradiated; however, attaching and propagation prevention may be performed using a device that continuously irradiates light (hereinafter called continuous irradiation).

For example, in a state fixing the irradiation device, light is continuously irradiated onto the rotating blades of a propeller or the like. At this time, by fixing the position of the propeller and the irradiation direction of light, and irradiating light so that light is irradiated onto part of the blades of the propeller, without irradiating light onto the entirety of the propeller from the irradiation device, it is possible to intermittently irradiate each blade of the propeller, while performing continuous irradiation from the irradiation device. Then, by adjusting the rotation speed of the propeller and light irradiance from the irradiation device and continuously irradiating so as to satisfy the aforementioned Formula (1), it is possible to efficiently suppress attachment of sessile organisms.

In addition, light may be continuously irradiated onto large-scale structures such as ships while anchored, while mounting the irradiation device to a drone or the like and moving so as to scan the ship. At this time, by causing the irradiation device to move so that light is irradiated regularly to the same locations on a ship, the same effects as intermittent irradiation are exhibited. Then, by adjusting the irradiance from the irradiation device and the movement speed of the irradiation device so as to satisfy the conditions expressed by Formula (1) and continuously irradiating, it is possible to efficiently suppress the attachment of sessile organisms. In addition, for example, under a single-pass seawater flow condition, it is possible to effectively suppress biofouling damage in a facility without negatively affecting the environment, by irradiating high intensity light at the upstream to suppress subsequent attachment for a fixed time in a downstream region, then draining, and restoring adhesion. Such an irradiation method can also be considered a series of intermittent irradiation.

EXAMPLES

Hereinafter, the contents of the present invention will be described in further detail based on the Examples. The contents of the present invention are not limited to the descriptions of the following Examples.

(Attaching Prevention Effect Confirmation Test 1)

An attaching prevention effect of organisms was confirmed by LED light using cypris larva of Balanus amphitrite (hereinafter referred to as cypris larva). It should be noted that cypris larva is larva in the adhesion period, reached by nauplius larva in the floating period repeating molting, and growing to be attachable to a target. Juvenile barnacles described later are barnacles reached by cypris larva attaching to a target such as a test container, and undergoing metamorphosis.

To a test container (cylinder of 6 cm diameter x 6 cm height), 70 ml of test seawater (seawater filtered through 0.45 $\mu$m pore-size membrane), and cypris larva (population: 44+/-12, provided 30+/-5 in test section of 1200 Wm$^{-2}$ irradiance) were added. As the test section, LED light (peak wavelength: 405 nm) was irradiated through a glass cover to this test container under the respective irradiation conditions shown in Tables 1 to 3 below. As the light source of the LED light, a UFL-501-08 UV-UT (manufactured by U-Technology Co., Ltd.) was used. In order to prevent a temperature rise of the test seawater or the like due to irradiation of LED light, in the test, the bottom outer side of the test container was immersed in tap water. The test container was covered with aluminum foil, and the portion establishing substantially constant dark conditions without irradiating LED light was set as the control section. The test was performed for 96 hours to compare the juvenile barnacle rate between the control section and each test section. It should be noted that juvenile barnacle rate is a proportion of juvenile barnacles accounted for in the Amphibalanus amphitrite population existing in each test container.

[Table 1]

| Irradiance (Wm$^{-2}$) | 100 | 200 | 200 | 200 | 200 | 0 (control section) |
|---|---|---|---|---|---|---|
| Lighting time (seconds) | Continuous | Continuous | 0.2 | 0.1 | 0.1 | - |
| Lights-out time (seconds) | - | - | 0.1 | 0.1 | 0.2 | - |
| N number | 15 | 15 | 3 | 3 | 3 | 15 |
| Juvenile barnacle rate average +/- after 96 hr (%) | 7.5±5.8 | 0.5±1.3 | 1.3±1.8 | 6.4±0.8 | 23.2±13.3 | 93.5±5.3 |

[Table 2]

| Irradiance (Wm$^{-2}$) | 400 | 600 | 600 | 600 | 600 | 600 | 0 (control section) |
|---|---|---|---|---|---|---|---|
| Lighting time (seconds) | 0.1 | 0.1 | 0.1 | 0.1 | 0.1 | 0.1 | - |
| Lights-out time (seconds) | 0.1 | 0.2 | 0.3 | 0.4 | 0.5 | 1.1 | - |
| N number | 15 | 12 | 11 | 9 | 18 | 3 | 25 |
| Juvenile barnacle rate average +/- after 96 hr (%) | 0.5±1.2 | 0.9±1.6 | 4.1±8.3 | 6.1±9.2 | 7.5±6.5 | 77.3±6.2 | 81.2±12.2 |

[Table 3]

| Irradiance (Wm$^{-2}$) | 600 | 1200 | 0 (control section) |
|---|---|---|---|
| Lighting time (minutes) | 5 | 12 | - |
| Lights-out time (minutes) | 10 | 60 | - |
| N number | 12 | 15 | 20 |

(continued)

| Irradiance (Wm$^{-2}$) | 600 | 1200 | 0 (control section) |
|---|---|---|---|
| Juvenile barnacle rate average +/- after 96 hr (%) | 0.7±1.0 | 1.8±2.7 | 89.3±4.5 |

(Test Results)

Table 1 shows the juvenile barnacle rate of the test sections at continuous irradiation of 100 Wm$^{-2}$ irradiance, continuous irradiation of 200 Wm$^{-2}$ irradiance, and intermittent irradiation of 200 Wm$^{-2}$ irradiance (0.2 seconds lighting / 0.1 seconds lights-out, 0.1 seconds lighting / 0.1 seconds lights-out, 0.1 seconds lighting / 0.2 seconds lights-out) and the control section. Table 2 shows the juvenile barnacle rate of the test section at intermittent irradiation of 400 Wm$^{-2}$ irradiance (0.1 seconds lighting / 0.1 seconds lights-out) and intermittent irradiation of 600 Wm$^{-2}$ irradiance (0.1 seconds lighting / 0.2 to 1.1 seconds lights-out) and the control section. Table 3 shows the juvenile barnacle rate of the test section at intermittent irradiation of 600 Wm$^{-2}$ irradiance (5 minutes lighting / 10 minutes lights-out) and intermittent irradiation of 1200 Wm$^{-2}$ irradiance (12 minutes lighting / 60 minutes lights-out) and the control section. N number in Table 1 is the number of times testing (same in following tables).

As shown in Table 1, at the irradiation conditions of 200 Wm$^{-2}$ irradiance for 0.1 seconds lighting / 0.1 to 0.2 seconds lights-out, it was confirmed that the attachment of barnacles could be drastically reduced compared to the control section. At the irradiation conditions of continuous irradiation and 0.2 seconds lighting/0.1 seconds lights-out, no leg movement was observed in any of the very few attached individuals, and it was confirmed that attachment to the test container is almost completely preventable.

As shown in Table 2, at irradiation conditions of 400 Wm$^{-2}$ irradiance for 0.1 seconds lighting / 0.1 seconds lights-out, and irradiation conditions of 600 Wm$^{-2}$ irradiance for 0.1 seconds lighting / 0.2 seconds lights-out, no leg movement was observed in any of the very few attached individuals, and it was confirmed that attaching to the test container is almost completely preventable. At the irradiation conditions of 600 Wm$^{-2}$ irradiance for 0.1 seconds lighting / 1.1 seconds lights-out, it was confirmed that, although there is a difference of about 0.4% compared to the control section, attaching of barnacles could be suppressed. At other irradiation conditions, it was confirmed that attaching of barnacles can be drastically reduced compared to the control section.

As shown in Table 3, at irradiation conditions of 600 Wm$^{-2}$ irradiance for 5 minute lighting / 10 minute lights-out, and irradiation conditions of 1200 Wm$^{-2}$ irradiance for 12 minute lighting / 60 minute lights-out, no leg movement was observed in the very few attached individuals, and it was confirmed that attaching to the test container is almost completely preventable.

As shown in Tables 1 to 3, the juvenile barnacle rate (0.5 +/- 1.3%) of continuous irradiation of 200 Wm$^{-2}$ irradiance, the juvenile barnacle rate (0.5 +/- 1.2%) of 400 Wm$^{-2}$ irradiation for 0.1 seconds lighting / 0.1 seconds lights-out, the juvenile barnacle rate (0.9 +/- 1.6%) of 600 Wm$^{-2}$ irradiation for 0.1 seconds lighting / 0.2 seconds lights-out, the juvenile barnacle rate (0.7 +/- 1.0%) of 600 Wm$^{-2}$ irradiation for 5 minutes lighting / 10 minutes lights-out, and the juvenile barnacle rate (1.8 +/- 2.7%) of 1200 Wm$^{-2}$ irradiation for 12 minutes lighting / 60 minutes lights-out each gave very similar results. In addition, similarly, the juvenile barnacle rate (7.5 +/- 5.8%) of continuous irradiation with 100 Wm$^{-2}$ irradiance, and juvenile barnacle rate (7.5 +/- 6.5%) of 600 Wm$^{-2}$ irradiance for 0.1 seconds lighting / 0.5 seconds lights-out gave very similar results.

(Significance in Test Results)

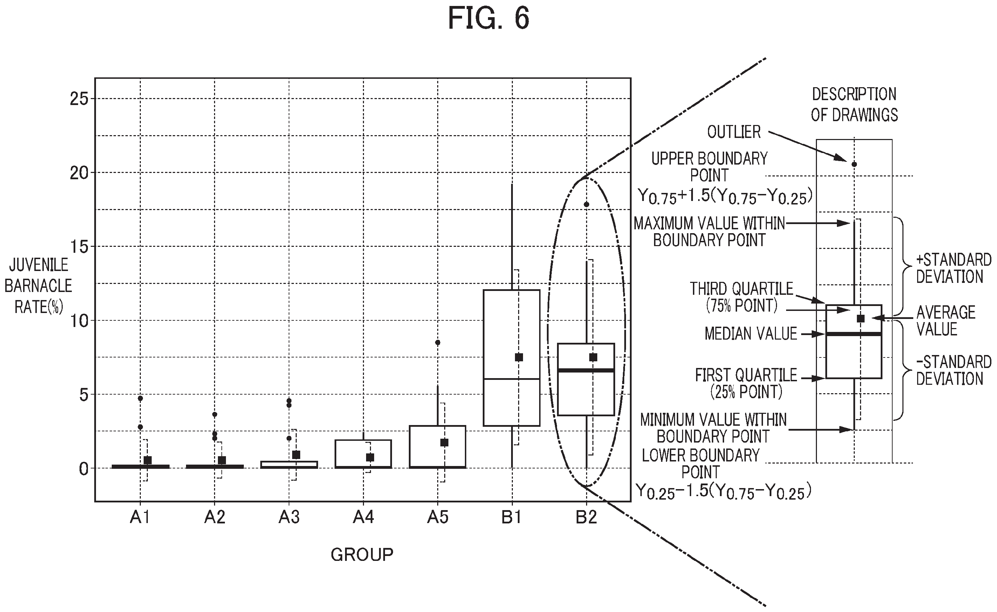

Herein, a significance test was performed in multiple comparison test (Tukey HSD method) by statistical software R on the results of juvenile barnacle rate at each irradiation condition of the Attaching Prevention Effect Confirmation Test 1. FIG. 6 is a boxplot related to the results of juvenile barnacle rate at each irradiation condition. The horizontal axis of FIG. 6 shows the respective irradiation conditions of the Attaching Prevention Effect Confirmation Test 1, and the vertical axis shows the juvenile barnacle rate (%). It should be noted that A1 indicates continuous irradiation with 200 Wm$^{-2}$ irradiance, A2 indicates intermittent irradiation with 400 Wm$^{-2}$ irradiance for 0.1 seconds lighting / 0.1 seconds lights-out, A3 indicates intermittent irradiation with 600 Wm$^{-2}$ irradiance for 0.1 seconds lighting / 0.2 seconds lights-out, A4 indicates intermittent irradiation with 600 Wm$^{-2}$ irradiance for 5 minutes lighting / 10 minutes lights-out, A5 indicates intermittent irradiation with 1200 Wm$^{-2}$ irradiance for 12 minutes lighting / 60 minutes lights-out, B1 indicates continuous irradiation with 100 Wm$^{-2}$ irradiance, and B2 indicates intermittent irradiation with 600 Wm$^{-2}$ irradiance for 0.1 seconds lighting / 0.5 seconds lights-out.

As a result of significance test, significant differences between the results of each juvenile barnacle rate in A1 to A5 were not observed (p value > 0.05, p valve 0.26 to 0.99). Similarly, significant differences between the results of each juvenile barnacle rate in B1 and B2 were not observed (p value > 0.05, p value 0.99).

From the results of the juvenile barnacle rates of A1 to A5, B1 and B2, and the results of the significance test, the required irradiation conditions for attachment prevention in each intermittent irradiation could be confirmed to be proportional to the irradiation energy. In other words, for the required irradiation conditions for organism attachment prevention in intermittent irradiation, it could be confirmed that the equation of aforementioned Formula (1) holds true.

Herein, it is proven that the attachment to a target and/or propagation of barnacle larva could be suppressed at continuous irradiation of 20 $Wm^{-2}$ irradiance (light wavelength peak: wavelength region of 400 to 430 nm), and attachment of barnacle larva to the target could be almost completely prevented with continuous irradiation of 150 $Wm^{-2}$ irradiance (light wavelength peak: wavelength region of 400 to 430 nm). When applying this knowledge, the irradiation conditions of light required in order to prevent attachment of barnacles in the case of intermittently irradiating light (light wavelength peak: wavelength region of 400 to 430 nm) could be obtained using the following Formula (2) or Formula (3).

$$X \times a/(a+b) \geqq 20 \ ... \ \text{Formula (2)}$$

$$X \times a/(a+b) \geqq 150 \ ... \ \text{Formula (3)}$$

(Phototaxis Confirmation Test)

Phototaxis of cypris larva was confirmed. LED light (peak wavelength: 405 nm) was continuously irradiated for 96 hours onto the cypris larva in a test container by the same procedure as the Attaching Prevention Effect Confirmation Test 1, excluding the irradiation condition of LED light. As a test section, tests were performed under the two conditions of irradiance of 20 $Wm^{-2}$ and 50 $Wm^{-2}$.

(Test Result)

Table 4 is a table showing the positional distribution of barnacles attached to a petri dish in the test section of 20 $Wm^{-2}$ irradiance and the control section thereof. Table 5 is a table showing the positional distribution of barnacles attached to a petri dish in the test section of 50 $Wm^{-2}$ irradiance and the control section thereof.

[Table 4]

| Irradiance ($Wm^{-2}$) | 0 (control section) | 20 |
|---|---|---|
| N number | 22 | 15 |
| Attachment ratio to lateral surface (%) | 18.8±7.3 | 46.0±12.4 |
| Attachment ratio to bottom surface (%) | 81.2±7.3 | 54.0±12.4 |

[Table 5]

| Irradiance ($Wm^{-2}$) | 0 (control section) | 50 |
|---|---|---|
| N number | 10 | 6 |
| Attachment ratio to lateral surface (%) | 20.8±8.6 | 44.7±19.3 |
| Attachment ratio to bottom surface (%) | 79.2±8.6 | 55.3±19.3 |

As shown in Table 4 and Table 5, at both conditions of 20 $Wm^{-2}$ irradiance and 50 $Wm^{-2}$ irradiance, about 80% of individuals attached to the bottom surface of the petri dish, and about 20% of individuals attached to the lateral surface of the petri dish in the control section. On the other hand, the difference in attachment ratio to the bottom surface and lateral surface of the petri dish in the test section could be confirmed to be small. As a result of performing multiple comparison tests (Bonferroni method) for lateral surface attachment rate in the control section and lateral surface attachment rate in the test section, it was confirmed that the lateral surface attachment rate to the petri dish in the test section increased significantly ($p < 0.01$). From this result, it is speculated that there is a tendency for the cypris larva to avoid locations where LED light (wavelength peak: 405 nm) directly irradiated, even when attached.

(Effect Confirmation Test for Phototaxis)

Using cypris larva of Amphibalanus amphitrite, the influence on the swimming behavior of cypris larva by LED light was confirmed.

To a test water tank (1-cm width x 1-cm height x 12-cm length), 20 ml of test seawater (seawater filtered through 0.45 $\mu$m pore-size membrane) was poured, and a light source irradiating LED light was arranged horizontally in the test water tank at an end in the length direction of the test water tank. As the light source, a UFL-501-08 UV-UT (manufactured by U-Technology Co., Ltd.) (wavelength peak: 405 nm) and a UFL-501-08 G-UT (manufactured by U-Technology Co., Ltd.) (wavelength peak: 520 nm) were used. To the test water tank, numbers 1 to 24 were attached as lane numbers at 5 mm intervals in ascending order from an end portion on the light source side in the length direction thereto. Cypris larva were added to lane number 12 of the test water tank filled with test seawater, and LED light was irradiated from the light source according to the respective irradiation conditions. The irradiation conditions are described later. It was confirmed to which lane number the cypris larva migrated after 1 minute from the start of irradiation. Fifteen individual cypris larva were used in one time testing, and five were placed in each test water tank, and observed for three times testing. This test was repeated three times for every irradiation condition. In other words, tests were performed for a total of 45 individuals for one irradiation condition.

(Irradiation Condition)

The irradiation condition of LED light was set to irradiance of 20 Wm$^{-2}$, 400 Wm$^{-2}$, 600 Wm$^{-2}$ or 2000 Wm$^{-2}$ at wavelength of wavelength peak 405 nm or wavelength of wavelength peak 520 nm, and phototaxis of cypris larva in continuous irradiation or the following intermittent irradiation was confirmed at conditions combining the respective wavelengths and irradiance.

<Intermittent Irradiation Condition>

When setting the lighting time (sec) / lights-out time (sec), five conditions of 0.1/0.1, 0.1/0.2, 0.1/0.3, 1/1, 1/2

(Test Results)

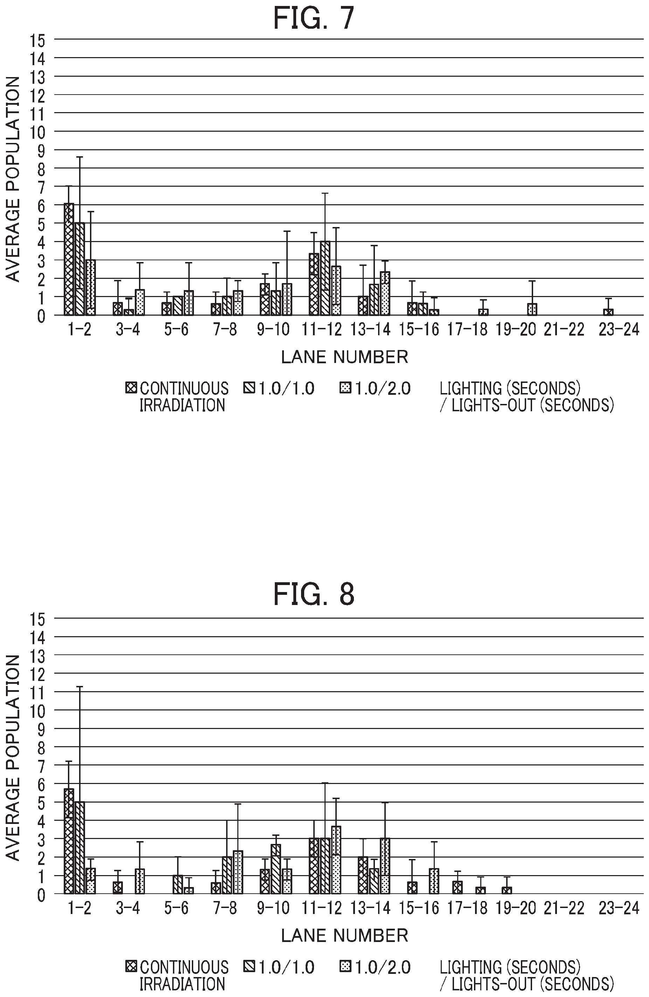

FIGS. 7 to 12 show the results of the present tests. The horizontal axis of FIGS. 7 to 12 is the lane number of the test water tank in which cypris larva were confirmed 1 minute after the start of irradiation, and the vertical axis is the average population of three tests of cypris larva migrated to each lane number.

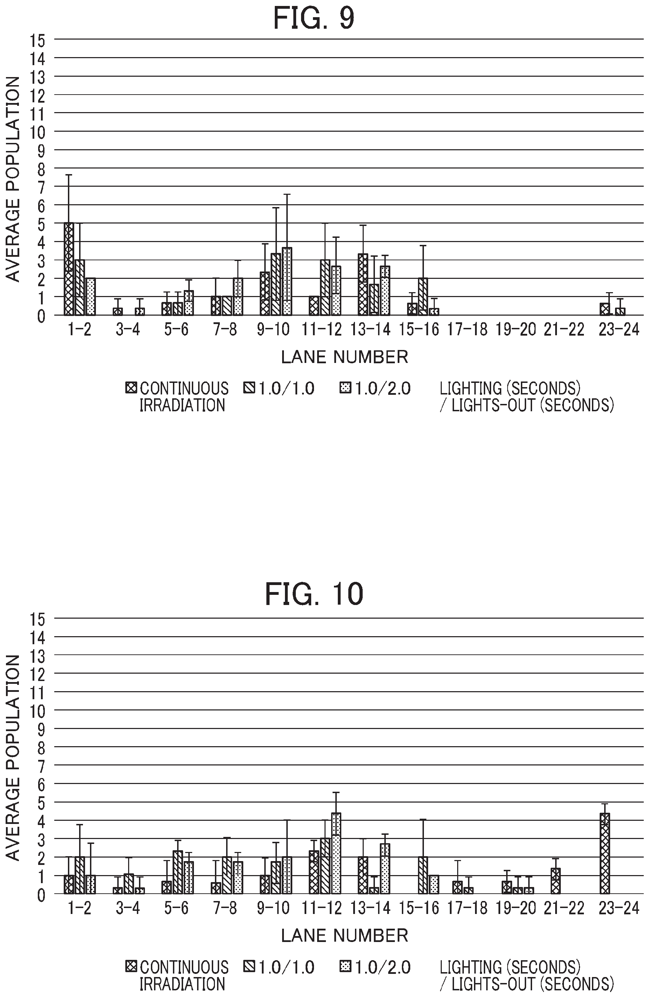

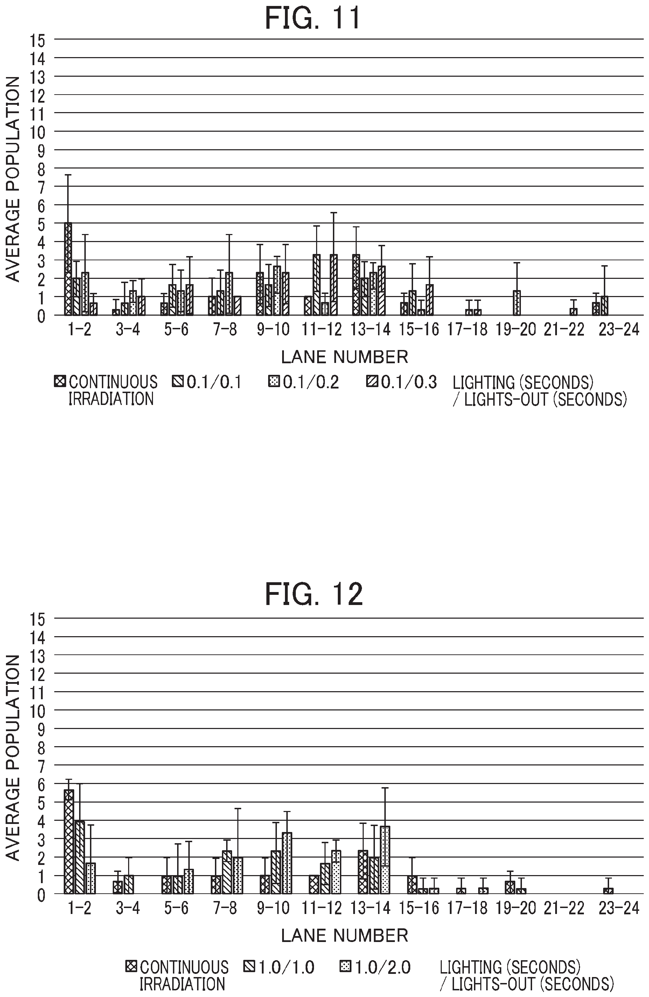

FIGS. 7 to 11 show the test results for the irradiation condition of 405 nm wavelength, and FIG. 12 shows the test results for the irradiation condition of 520 nm wavelength. In addition, FIG. 7 is the irradiation condition of continuous irradiation and intermittent irradiation (1/1, 1/2) for 20 Wm$^{-2}$ irradiance, FIG. 8 is the irradiation condition of continuous irradiation and intermittent irradiation (1/1, 1/2) for 400 Wm$^{-2}$ irradiance, FIG. 9 is the irradiation condition of continuous irradiation and intermittent irradiation (1/1, 1/2) for 600 Wm$^{-2}$ irradiance, FIG. 10 is the irradiation condition of continuous irradiation and intermittent irradiation (1/1, 1/2) for 2000 Wm$^{-2}$ irradiance, FIG. 11 is the irradiation condition of continuous irradiation and intermittent irradiation (0.1/0.1, 0.1/0.2, 0.1/0.3) for 600 Wm$^{-2}$ irradiance, and FIG. 12 is the irradiation condition of continuous irradiation and intermittent irradiation (1/1, 1/2) for 600 Wm$^{-2}$ irradiance,

As shown in FIGS. 7 to 11, it was confirmed to have a trend of cypris larva approaching the light source, and a trend of swimming in the opposite direction, according to the continuous irradiation of LED light of 405 nm wavelength. In particular, with continuous irradiation of 2000 Wm$^{-2}$ irradiance, clear avoidance/escape behavior of swimming in the opposite direction as the light source was confirmed. On the other hand, in the case of intermittent irradiation, a trend was confirmed whereby the individuals attracted to the light source decreased compared to continuous irradiation, and the individuals avoiding the light source in the opposite direction as the light source also decreased. From this result, it is speculated that the movement due to phototaxis of cypris larva relative to irradiation of light is suppressed by intermittent irradiation.

As shown in FIG. 12, it was confirmed that many larva swam to the light source due to continuous irradiance at 520 nm wavelength. On the other hand, in the case of intermittent irradiation, it was confirmed that the individuals approaching the light source decreased compared to continuous irradiation. In other words, it is speculated that attraction caused by phototaxis of cypris larva relative to light of 520 nm wavelength is suppressed by intermittent irradiation.

(Attaching Prevention Effect Confirmation Test 2)

The attaching prevention effect from the irradiation of light on hydrozoans was confirmed using a hydra stem segment of Coryne pusilla which is a hydrozoan. It should be noted that the hydra stem segments were prepared by isolating the polyps from a Coryne pusilla colony, and cutting off the hydra roots and hydra flower.

To a test container (glass petri dish of 6 cm diameter x 6 cm height), 70 ml of test seawater (seawater filtered through 0.45 $\mu$m pore-size membrane) and hydra stem segments (count: 12 segments) were added. As a test section, LED light (peak wavelength: 405 nm) was irradiated through a glass cover to this test container under the respective irradiation conditions shown in Tables 4 and 5 below. The test container was covered with aluminum foil, and the portion establishing substantially constant dark conditions without irradiating LED light was defined as the control section. The test was performed for 96 hours to compare the regeneration rate of hydra stem fragments and regenerated hydra flower number between the control section and each test section.

[Table 6]

| Irradiance (Wm$^{-2}$) | Lighting time (h) | Lights-out time (h) | N number | Regeneration rate average +/- SD after 96 hr (%) | Hydra flower number average +/-SD after 96 hr (%) |
|---|---|---|---|---|---|
| 200 | 1 | 1 | 3 | 0.0±0.0 | 0.0±0.0 |
| 0 (control section) | - | - | 5 | 91.4±8.7 | 14.4±2.7 |

[Table 7]

| Irradiance (Wm$^{-2}$) | Lighting time (h) | Lights-out time (h) | N number | Regeneration rate average +/- SD after 96 hr (%) | Hydra flower number average +/-SD after 96 hr (%) |
|---|---|---|---|---|---|
| 200 | 2 | 2 | 3 | 0.0±0.0 | 0.0±0.0 |
| 0 (control section) | - | - | 5 | 100.0±0.0 | 17.4±1.9 |

(Test Results)

Table 6 shows the regeneration rate of hydra stem segments and regenerated hydra flower number after 96 hours in the test section of irradiation conditions of intermittent irradiation (1 hr lighting / 1 hr lights-out) with 200 Wm$^{-2}$ irradiance, and the control section. Table 7 shows the regeneration rate of hydra stem segments and regenerated hydra flower number after 96 hours in the test section of irradiation conditions of intermittent irradiation (2 hr lighting / 2 hr lights-out) with 200 Wm$^{-2}$ irradiance, and the control section. As shown in Table 6 and Table 7, at the two intermittent irradiation conditions of 200 Wm$^{-2}$ irradiance, it was confirmed that the regeneration rate of hydra stem segments and regenerated hydra flower number after 96 hours was 0%. In addition, the stem tissue of Coryne pusilla gradually regressed from the cut portion in the test, and a crumbling state was observed. From this result, it could be confirmed that colony formation of marine hydrozoans could be completely prevented at the two conditions of intermittent irradiation conditions (1 hr lighting / 1 hr lights-out) (2 hr lighting / 2 hr lights-out) with 200 Wm$^{-2}$ irradiance. It should be noted that, compared to the results of Attaching Prevention Effect Confirmation Test 1, it was observed that hydrozoans had lower resistance to indigo light of 405 +/- 25 nm wavelength than barnacles.

(Attaching Prevention Effect Confirmation Test 3)

The influence from light irradiation on freshwater hydra was confirmed. To a 12-hole well plate, 3 ml of test water was added, one freshwater hydra was added for each well, and after confirming that the tentacles and body had sufficiently extended, LED light (peak wavelength: 405 nm) was irradiated at the respective irradiation conditions described later, obliquely from above the well plate. The irradiation time was set to 3 minutes, and whether the freshwater hydra contracted or shrink the body was observed under a microscope. Based on the tests of each irradiation condition, fifteen individuals were observed.
The test water was prepared by adding 2 ml of each of the below solutions (a) to (c) respectively to 1 L of pure water.

(a) 0.5 M CaCl$_2$, 0.5 M NaCl, 0.05 M KCl
(b) 0.05 M MgSO$_4$
(c) 0.5 M Tris HCl (pH 7.5)

As the irradiation conditions of LED light, the irradiance was set to 500 Wm$^{-2}$ or 1000 Wm$^{-2}$, and a plurality of intermittent irradiation conditions were set for each irradiance. The intermittent irradiation condition fixes the lighting time to 0.1 seconds, and sets the lights-out time as follows for every irradiance.

<Lights-out time for 500 Wm$^{-2}$>

0 seconds (i.e. continuous irradiation), 0.1 seconds, 0.2 seconds, 0.3 seconds, 0.4 seconds, 0.5 seconds, 0.6 seconds, 0.7 seconds, 0.8 seconds, 0.9 seconds, 1.0 seconds

<Lights-out time for 1000 Wm$^{-2}$>

0 seconds (i.e. continuous irradiation), 0.8 seconds, 0.9 seconds, 1.0 seconds, 1.3 seconds, 1.5 seconds, 2.0 seconds, 3.0 seconds

(Test Results)

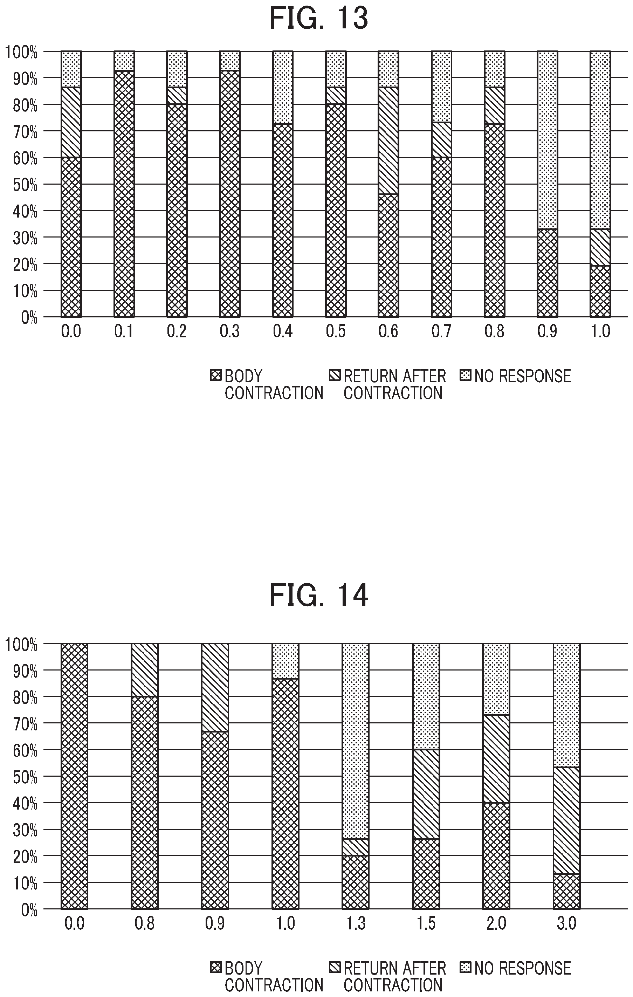

FIG. 13 shows the evaluation of reaction status of freshwater hydra from LED light irradiation at the respective intermittent irradiation conditions when 500 Wm$^{-2}$ irradiance. FIG. 14 shows the evaluation of reaction status of freshwater hydra from LED light irradiation at the respective intermittent irradiation conditions when 1000 Wm$^{-2}$ irradiance. The horizontal axis in FIG. 13 and FIG. 14 shows the lights-out time for each intermittent irradiation condition, and the vertical axis shows the proportion of the total occupied by freshwater hydra exhibiting each reaction. The reaction status was evaluated as "body contraction" indicating that the body of the freshwater hydra contracted, "return after contraction" indicating returning after body contraction, and "no response" indicating not responding to LED light irradiation. It should be noted that FIG. 13 and FIG. 14 are showing "body contraction" with the densest dot pattern, "return after contraction" with the next densest dot pattern, and "no response" with the sparsest dot pattern.

As shown in FIG. 13, it could be confirmed that 80% or more of individuals exhibited a reaction of contracting the body with lighting time 0.1 seconds / lights-out time 0.1 to 0.3 seconds when 500 Wm$^{-2}$ irradiance. In addition, it could be confirmed that 80% or more of individuals exhibited a reaction of contracting the body with lighting time 0.1 seconds / lights-out time 0.8 to 1.0 seconds when 1000 Wm$^{-2}$ irradiance. On the other hand, it could be confirmed that the reaction rate of body contraction decreases when the lights-out time became 1.3 seconds or more. From these results, it is speculated that, also for freshwater hydra, attaching to the target could be suppressed similarly to continuous irradiation, by predetermined intermittent irradiation conditions set according to the above Formula (2), etc.

(Attaching Prevention Effect Confirmation Test 4)

The influence of light irradiation on bacterial growth was confirmed by smearing marine bacteria onto an agar medium, followed by counting the number of colonies appearing after irradiation of LED light.

Onto the agar medium surface in a petri dish (9 cm diameter x 1.5-cm height), 0.1 ml of seawater containing marine bacteria (all types of marine bacteria in sea water in barnacle breeding aquarium) was dropped and smeared, and half of the smeared agar medium was covered with aluminum foil to establish a control section. Then, LED light (peak wavelength: 405 nm) was irradiated under the respective irradiation conditions shown in Table 8 from above the petri dish, and the colony numbers in the control section and test section on each agar medium surface after about 24 hours and after about 48 hours from the start of irradiation were counted. This was repeated three times, and the appearance ratio of colony numbers in the irradiation section relative to the colony number of the control section was calculated.

As the agar medium, powdered culture medium (Marine Agar 2216, manufactured by Difco) was added to 1 L of distilled water, and after heating to dissolve and sterilize, about 20 ml was dispensed to the petri dish, left to stand for 30 to 60 minutes, and used as agar solid growth medium. As the seawater smeared on the agar medium, seawater filtered through a 0.45 $\mu$m pore-size membrane and diluted 10 times was used.

[Table 8]

| Irradiation conditions | | | | 1st time | 2nd time | 3b time | Average |
|---|---|---|---|---|---|---|---|
| Irradiance | Lighting time / lights-out time | Irradiation time | Elapsed time after irradiation start | Bacterial colony appearance ratio (irradiation section colony number / control section colony number) | Bacterial colony appearance ratio (irradiation section colony number / control section colony number) | Bacterial colony appearance ratio (irradiation section colony number / control section colony number) | Bacterial colony appearance ratio average +/- SD |
| 200 Wm$^{-2}$ | Continuous | 8h | 24h | 0% (0/126) | 2.2% (3/138) | 0% (0/199) | 0.7±1.27% |
| 200 Wm$^{-2}$ | Continuous | 8h | 48h | 1.4% (2/141) | 2.1% (3/141) | 6.5% (13/200) | 3.3±2.76% |
| 400 Wm$^{-2}$ | 1min/1min | 8h | 24h | 0% (0/107) | 3.0% (5/167) | 0% (0/150) | 1.0±1.73% |
| 400 Wm$^{-2}$ | 1min/1min | 8h | 48h | 3.2% (4/124) | 3.4% (6/175) | 8.6% (13/152) | 5.1±3.06% |
| 400 Wm$^{-2}$ | 1h/1h | 8h | 24h | 0% (0/124) | 3.6% (5/140) | 0% (0/215) | 1.2±2.07% |
| 400 Wm$^{-2}$ | 1h/1h | 8h | 48h | 0% (0/138) | 3.2% (5/157) | 6.4% (14/218) | 3.2±3.2% |

(Test Results)

Table 8 shows the appearance ratio (hereinafter referred to as bacterial colony appearance ratio) of colony number of irradiation group relative to colony number of control section in the case of continuous irradiation for 8 hours at 200 Wm$^{-2}$ irradiance, the case of intermittent irradiation (1 minute lighting/1 minute lights-out) for 8 hours at 400 Wm$^{-2}$ irradiance, and the case of intermittent irradiation (1 hr lighting / 1 hr lights-out) for 8 hours at 400 Wm$^{-2}$ irradiance. It should be noted that the irradiation quantity of LED light for the three irradiation conditions were all equal at 1600 Whm$^{-2}$.

As shown in Table 8, the bacterial colony appearance ratio at all irradiation conditions was lower than 10% even after 48 hours, and a similar bacteria effect to continuous irradiation was confirmed for intermittent irradiation on marine bacteria. In addition, the bacteria colony appearance ratio for all irradiation conditions gave substantially the same result, whereby it could be confirmed that, so long as the irradiation quantity is identical, the same propagation prevention effect will be exhibited.

(Attachment Test 1)

The test apparatus was installed by suspending in the sea by metal chain or rope. The installation position is a position of about 2 m depth. The test apparatus fixed two test plates (60 cm x 60 cm x 5 mm thick, made of vinyl chloride, evaluated surface 50 cm x 50 cm) arranged with an interval, and underwater LED lamps (peak wavelength: 407 nm) arranged at intervals between the two test plates, and irradiating LED light onto the two test plates. The distance between the irradiation surface of the underwater LED lamps and one test plate was set to about 20 cm, and the distance between the irradiation surface of the underwater LED lamps and the other test plate was set to about 30 cm. As a control section, the test apparatus was installed in the sea without arranging underwater LED lamps therein. The test period was set to about 1 month from April 14 to May 18, 2022. The test was performed according to the following six conditions.

Example 1: Using underwater LED lamps (irradiance: 200 Wm$^{-2}$ (at center position of evaluation surface of test plate), interval from test plate: 30 cm), switching between irradiation and non-irradiation was performed every 1 second to intermittently irradiate towards the center of the test plate. Example 2: Using underwater LED lamps (irradiance: 400 Wm$^{-2}$ (at center position of evaluation surface of test plate), interval from test plate: 20 cm), switching between irradiation and non-irradiation was performed every 1 second to intermittently irradiate towards the center of the test plate. Comparative Example 1: Using underwater LED lamps (irradiance: 200 Wm$^{-2}$ (at center position of evaluation surface of test plate) interval from test plate: 30 cm), continuous irradiation toward center of test plate was performed. Comparative Example 2:

Using underwater LED lamps (irradiance: 400 Wm$^{-2}$ (at center position of evaluation surface of test plate) interval from test plate: 20 cm), continuous irradiation toward center of test plate was performed. Comparative Example 3: It was left in the sea without using underwater LED lamps (test surface on building side). Comparative Example 4: It was left in the sea without using underwater LED lamps (test surface on pier side).

(Test Results)

Table 9 and Table 10 are tables showing organism attaching status on the surface of the test plate of the control section. Table 9 shows the attachment number of every organism attached to the surface of the test plate, the colony number analysis result (ascidians, tunicates are covered area). Table 10 shows the wet weight analysis result for every organism attached to the surface of the test plate (measuring each organism after peeling off).

[Table 9]

| Irradiation conditions | | Attachment weight to test plate surface by each organism (cm$^2$) | | | | | | | | |
|---|---|---|---|---|---|---|---|---|---|---|
| Illumination | Immersion position | Ascidians | Tunicates | Colonial sea squirts | Muddy amphipod tubes | Itakokemushi | Watersipora subovoidea | Bugula neritina | Spirorbidae | Sabellidae |
| 0 | Building side | 142.25 | 2 | 5 | 0 | 23 | 0 | 13 | 4 | 0 |
| 0 | Pier side | 10 | 0 | 0 | 528 | 31 | 0 | 1 | 1 | 0 |

EP 4 775 032 A1

[Table 10]

| Irradiation conditions | | Attachment weight to test plate surface by each organism (g) | | | | | | |
|---|---|---|---|---|---|---|---|---|
| Illumination | Immersion position | Ascidians | Tunicates | Colonial sea squirts | Muddy amphipod tubes | Itakokemushi Watersipora subovoidea | Bugula neritina | Spirorbidae Sabellidae |
| 0 | Building side | 8.82 | <0.01 | <0.01 | 0 | 0.19 | 0.11 | 0.01 |
| 0 | Pier side | 0.16 | 0 | 0 | 13.87 | 0.24 | <0.01 | <0.01 |

In Comparative Example 3 and Comparative Example 4, a state was observed in which several of algae, colonial tunicates, muddy amphipod tubes, bryozoans, etc. had attached and are were covering the entire surface of the test plate. On the other hand, the attachment of organisms to the surface of the test plate was not observed at least by the naked eye, including the peripheral portion, in either of Examples 1 and 2, similarly to Comparative Examples 1 and 2. For this reason, even in the case of switching between irradiation and non-irradiation of 200 Wm$^{-2}$ light every 1 second to perform intermittent irradiation, the result of being able to prevent attachment of organisms was confirmed.

(Attachment Test 2)

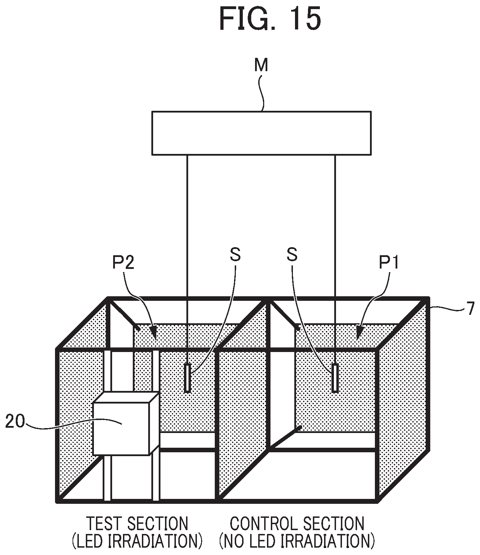

The test apparatus shown in FIG. 15 was installed by suspending by crane in the sea in the vicinity of a water intake of a power plant. The installation position was adjusted so that part of the test apparatus was not exposed above the sea even during spring-tide low tide, so as to be 1 m under the lowest water level. The test apparatus, as shown in FIG. 15, fixed the test plate P1 and test plate P2 (made of vinyl chloride, 25 cm x 25 cm), as well as the underwater LED irradiation device (peak wavelength: 405 nm, manufactured by DELPHIS) irradiating LED light onto the test plate P2 as a test section to the frame 7. The distance between the irradiation surface of the underwater LED irradiation device and the test plate P2 was set to about 35 cm. A control section was established by not irradiating light onto the test plate P1. A detector S of each water temperature sensor was arranged at the center of the test plates P1 and P2, and the change in water temperature were recorded by a recording unit M. The test period was set to about 1 month from August 19 to September 14, 2021. As the test section, tests were performed using the following three types of LED devices.

Example 3: A small-scale LED irradiation device (6 lamp type, irradiance setting: (at position of test plate surface) 100 Wm$^{-2}$) was used to perform switching between irradiation and non-irradiation every 15 minutes by a lab timer to intermittently irradiate towards the center of the test plate P2. Comparative Example 5: Using a small-scale LED irradiation device (6 lamp type, irradiance setting: (at position of test plate surface) 100 Wm$^{-2}$), continuous irradiation towards the center of the test plate P2 was performed. Comparative Example 6: Using a large-scale LED irradiation device (36 lamp type, irradiance setting: (at position of test plate surface) 100 Wm$^{-2}$), continuous irradiation towards the center of the test plate P2 was performed.

(Test Results)

Also for the test plates P2 in any of Example 3 and Comparative Examples 5 and 6, it was confirmed that attaching of foreign matter such as sessile organisms in the vicinity of a central portion of the test plate P2 on which LED light was irradiated was greatly suppressed, compared to the test plate P1 of the control section. On the other hand, attachment of green algae was confirmed at the periphery of the central portion of the test plates P2 on which LED light was irradiated, and the irradiation surface of the irradiation device in Comparative Examples 5 and 6. Almost no attachment of the above-mentioned green algae was observed on the test plate P2 of Example 3.

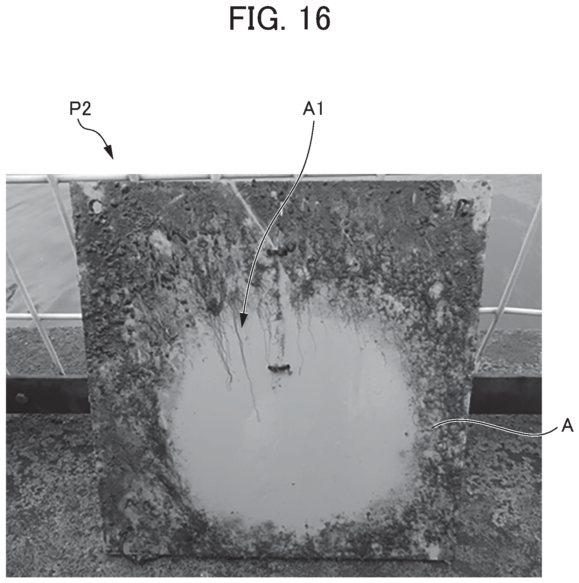

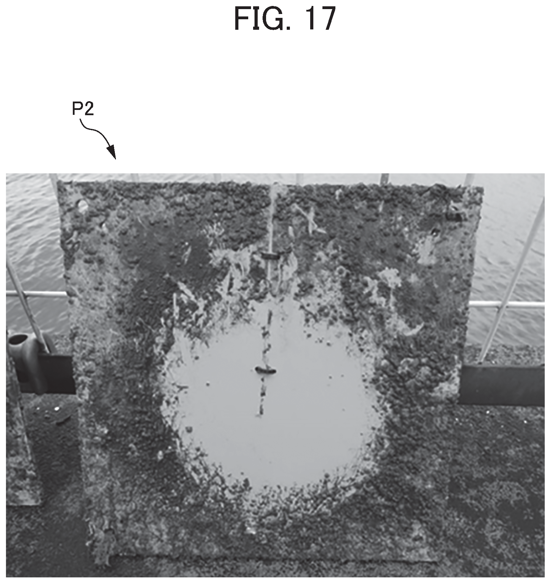

FIG. 16 is an image capturing the foreign matter attachment state of the test plate P2 of Comparative Example 6 after the attachment test. Green algae A and filamentous green algae A1 attached at the periphery of the central portion of the test plate P2 on which LED light was irradiated in Comparative Example 6. FIG. 17 is an image capturing the foreign matter attachment state of the test plate P2 of Example 3 after the attachment test. The attachment of green algae was not confirmed at the periphery of the central portion of the test plate P2 on which LED light was irradiated in Example 3. Therefore, the result was confirmed that not only the attachment of foreign matter such as sessile organisms to the irradiated location of LED light onto the target, but also the attachment of green algae to a peripheral region of an irradiated location can be suppressed by intermittently irradiating LED light of a predetermined wavelength.

Regarding the water temperature change detected by the water temperature sensor, a rise in water temperature of about 0.4°C compared to the control section was observed in the small-scale LED irradiation device used in Comparative Example 5, and a rise in water temperature of about 0.6°C compared to the control section was observed in the large-scale LED irradiation device used in Comparative Example 6. On the other hand, in Example 3, when switching irradiation to non-irradiation, it was confirmed that the water temperature quickly became equal to the control section.

For the small-scale LED irradiation device after the attachment test in Comparative Example 5, the irradiance after the attachment test dropped from 100 $Wm^{-2}$ to 58.2 $Wm^{-2}$. For the small-scale LED irradiation device after the attachment test in Comparative Example 6, the irradiance after the attachment test dropped from 100 $Wm^{-2}$ to 22.5 $Wm^{-2}$. When cleaning the irradiation surface of the LED irradiation device in Comparative Example 5 and Comparative Example 6, and measuring the irradiance again, the irradiance recovered to the neighborhood of 100 $Wm^{-2}$. For this reason, it is speculated that the drop in irradiance of Comparative Example 5 and Comparative Example 6 is due to the attachment of foreign matter to the irradiation surface. On the other hand, for the small-scale LED irradiation device of Example 3, a drop in the irradiance after the attachment test was not confirmed. For this reason, , the result was confirmed that the attachment of foreign matter to the irradiation surface of the LED irradiation device can be suppressed by intermittently irradiating LED light of a predetermined wavelength.

(Influence of Light Irradiation on Microorganisms in Air Confirmation Test 1)

By culturing microorganisms dropped onto LB medium, and counting the colony number appearing after the irradiation of LED light, the influence from light irradiation on the microorganisms attaching to a structure in air was confirmed.

LB medium was prepared by autoclave treatment of a mixture made by adding and mixing 5 g of tryptone, 2.5 g of yeast extract, 5 g of NaCl, 7.5 g of agar, and 480 ml of distilled water to a medium bottle, for 20 minutes at 121°C, and dispensing 20 ml of this onto a petri dish and allowing to solidify at room temperature. In a state with the lids of the three petri dishes in which this LB medium is placed left open, they were placed on a counter about 90 cm height above the floor within a room in the vicinity of the exit to outdoors, and left for 8 hours. The lids were closed after 8 hours, the three petri dishes were respectively labeled Samples 1 to 3, LED light (peak wavelength: 405 nm) was irradiated under the irradiation conditions of each sample shown in Table 11 from ascending order of each sample to culture each sample for 48 hours in a constant temperature incubator set to 35°C, 8 hours after irradiation start, and the colony number on the medium surface of each sample was counted. As the light source of LED light, a UFL-501-08 UV-UT (manufactured by U-Technology Co., Ltd.) was used. It should be noted that Sample 3 which is a petri dish left for 8 hours without irradiating LED light was defined as a control section.

[Table 11]

| Sample | Irradiation conditions | | | | Colony number | Sterilization rate relative to control section (%) |
|---|---|---|---|---|---|---|
| | Irradiance | Lighting time / lights-out time | Irradiation time | Irradiation quantity | | |
| 1 | 200$Wm^{-2}$ | Continuous | 8h | 1600$Whm^{-2}$ | 1 | 97.9 |
| 2 | 400W $m^{-2}$ | 1h / 1h | 8h | 1600$Whm^{-2}$ | 3 | 93.6 |
| 3 | 0 (control section) | - | - | 0 | 47 | - |

(Test Results)

Table 11 shows the colony number of each sample, and the sterilization rate relative to control section in the case of continuously irradiating and the case of intermittently irradiating LED light of 1600 $Whm^{-2}$ irradiation quantity. The sterilization rate relative to the control section shown in Table 11 is a value obtained by dividing the colony number of Sample 1 or 2 after irradiating LED light by the colony number in the control section of Sample 3. As shown in Table 11, in Sample 3 of the control section, 47 colonies of bacteria or fungi were observed. In contrast, one colony was observed in Sample 1 and three colonies were observed in Sample 2, and by irradiating LED light by intermittent irradiation with the irradiance Y in the above Formula (1) of 200 $Wm^{-2}$, the above sterilization rate reached 90% or higher. From this result, it is speculated that, also for sessile organisms that attach to the surface of structures in air such as airborne bacteria and fungi, the attaching and/or propagation of sessile organisms attaching to a structure in air can be almost completely prevented by adjusting so that the irradiance Y becomes 200 $Wm^{-2}$ or more.

(Influence of Light Irradiation to Microorganisms in Air Confirmation Test 2)

By culturing microorganisms dropped onto LB medium, and counting the colony number appearing after the irradiation of LED light at a lower irradiance than the irradiation condition shown in Table 12, the influence from light irradiation on the microorganisms attaching to a structure in air was confirmed.

The LB medium was prepared by the same procedure as the above "Influence of Light Irradiation on Microorganisms in Air Confirmation Test 1", and in a state with the lids of five petri dishes in which this LB medium is placed left open, they were placed on a counter about 90 cm height above the floor within a room in the vicinity of the exit to outdoors, and left for 8 hours. The lids were closed after 8 hours, the five petri dishes were respectively labeled Samples 4 to 8, LED light (peak wavelength: 405 nm) was irradiated under the irradiation conditions of each sample shown in Table 14 from ascending order of each sample to culture each sample for 48 hours in a constant temperature incubator set to 35°C, 8 hours after irradiation start, and the colony number on the medium surface of each sample was counted. As the light source of the LED light, a UFL-501-08 UV-UT (manufactured by U-Technology Co., Ltd.) was used. It should be noted that Samples 6 to 8 which are petri dish left for 8 hours without irradiating LED light were defined as a control section.

[Table 12]

| Sample | Irradiation conditions | | | | Colony number | Average colony number of Samples 6 to 8 | Sterilization rate relative to average colony number of control section (%) |
|---|---|---|---|---|---|---|---|
| | Irradiance | Lighting time / lights-out time | Irradiation time | Irradiation quantity | | | |
| 4 | $20 Wm^{-2}$ | Continuous | 8h | $160 Whm^{-2}$ | 5 | - | 66.7 |
| 5 | $40 Wm^{-2}$ | 1h / 1h | 8h | $160 Whm^{-2}$ | 5 | - | 66.7 |
| 6 | 0 (control section) | - | - | 0 | 18 | 15 | - |
| 7 | 0 (control section) | - | - | 0 | 13 | | - |
| 8 | 0 (control section) | - | - | 0 | 15 | | - |

(Test Results)

Table 12 shows the colony number of each sample, and a sterilization rate relative to an average colony number of a control section in the case of continuously irradiating and the case of intermittently irradiating LED light of 160 $Whm^{-2}$ irradiation quantity. The sterilization rate relative to the average colony number of the target group shown in Table 12 is a value obtained by dividing the colony number of Sample 4 or 5 after irradiating LED light by the average of the colony numbers in the control section of Samples 6 to 8. As shown in Table 12, in the three control sections, 13 to 18 colonies of bacteria or fungi were observed. In contrast, five colonies were observed in each of Samples 4 and 5, and even in the case of irradiating LED light by intermittent irradiation with the irradiance Y in the above Formula (1) of 20 $Wm^{-2}$, the above sterilization rate reached 60% or higher. From this result, it is speculated that, for sessile organisms that attach to the surface of structures in air such as airborne bacteria and fungi, attaching and propagation of sessile organisms to a target can be effectively prevented by adjusting so that the irradiance Y becomes 20 $Wm^{-2}$ or more.

EXPLANATION OF REFERENCE NUMERALS

1, 1a, 1b, 1c water quality meter
10 attaching and propagation prevention device
20, 20a, 20b, 20c, 20d, 20e irradiation device
22a, 22b, 22c, 22d, 22e irradiation surface
31, 32, 33, 34, 35, 36, 37, 38 detector
5, 5a, 5b, 5c controller

**Claims**

1. An attaching and propagation prevention method for suppressing attaching and/or propagation of a sessile organism to a target to which the sessile organism can attach, the method comprising:

    irradiating light onto the target intermittently to satisfy a condition expressed by Formula (1) below,

$$X \times a/(a+b) \geq Y \; ... \; \text{Formula (1)}$$

wherein X is irradiance of light $(Wm^{-2})$, a is a time for which light is irradiated, b is a time for which light is not irradiated, and Y is an irradiance of light $(Wm^{-2})$ set for every sessile organism.

2. The attaching and propagation prevention method according to claim 1, wherein Y in the Formula (1) is 20.

3. The attaching and propagation prevention method according to claim 1, wherein Y in the Formula (1) is 150.

4. The attaching and propagation prevention method according to any one of claims 1 to 3, wherein light irradiated onto the target has a peak in a wavelength region of 400 to 430 nm.

5. An attaching and propagation prevention device comprising: an irradiation device that irradiates light onto a target to which a sessile organism attaches; and a controller that controls the irradiation device, wherein

   the controller controls the irradiation device so that light is intermittently irradiated from the irradiation device onto the target satisfying a condition expressed by Formula (1) below,

$$X \times a/(a+b) \geq Y \; ... \; \text{Formula (1)}$$

   wherein X is irradiance $(Wm^{-2})$ of light from the irradiation device, a is a time for which light is irradiated from the irradiation device, b is a time for which light is not irradiated from the irradiation device, and Y is irradiance of light $(Wm^{-2})$ set for every sessile organism.

6. A water quality meter comprising the attaching and propagation prevention device according to claim 4; and a sensor device, wherein

   the irradiation device irradiates light onto a detector of the sensor device,
   the light irradiated from the irradiation device has a peak in a wavelength region of 400 to 430 nm,
   the sensor device performs a detection operation at every predetermined detection timing set in advance, and
   the controller controls the irradiation device so that light is intermittently irradiated onto the detector from the irradiation device every irradiation time outside the predetermined detection timing.

7. The water quality meter according to claim 6, wherein an irradiation surface of the irradiation device is disposed to oppose the detector.

# FIG. 1

10

| 20 | | 5 |
|---|---|---|
| IRRADIATION DEVICE | | CONTROLLER |

# FIG. 2

EP 4 775 032 A1

FIG. 3

# FIG. 4

EP 4 775 032 A1

# FIG. 5

# FIG. 6

JUVENILE BARNACLE RATE(%)

GROUP

DESCRIPTION OF DRAWINGS

OUTLIER

UPPER BOUNDARY POINT
$Y_{0.75}+1.5(Y_{0.75}-Y_{0.25})$

MAXIMUM VALUE WITHIN BOUNDARY POINT

+STANDARD DEVIATION

THIRD QUARTILE (75% POINT)

AVERAGE VALUE

MEDIAN VALUE

FIRST QUARTILE (25% POINT)

−STANDARD DEVIATION

MINIMUM VALUE WITHIN BOUNDARY POINT
LOWER BOUNDARY POINT
$Y_{0.25}-1.5(Y_{0.75}-Y_{0.25})$

EP 4 775 032 A1

## FIG. 7

## FIG. 8

# FIG. 9

# FIG. 10

# FIG. 11

AVERAGE POPULATION

LANE NUMBER

⊠ CONTINUOUS IRRADIATION   ◩ 0.1/0.1   ⊞ 0.1/0.2   ▨ 0.1/0.3   LIGHTING (SECONDS) / LIGHTS-OUT (SECONDS)

# FIG. 12

AVERAGE POPULATION

LANE NUMBER

⊠ CONTINUOUS IRRADIATION   ◩ 1.0/1.0   ⊞ 1.0/2.0   LIGHTING (SECONDS) / LIGHTS-OUT (SECONDS)

## FIG. 13

BODY CONTRACTION    RETURN AFTER CONTRACTION    NO RESPONSE

## FIG. 14

BODY CONTRACTION    RETURN AFTER CONTRACTION    NO RESPONSE

# FIG. 15

TEST SECTION
(LED IRRADIATION)　　　CONTROL SECTION
(NO LED IRRADIATION)

# FIG. 16

# FIG. 17

P2

## INTERNATIONAL SEARCH REPORT

| International application No. |
| --- |
| **PCT/JP2023/032193** |

| A. | CLASSIFICATION OF SUBJECT MATTER |
| --- | --- |

*A01M 29/30*(2011.01)i; *A01M 29/10*(2011.01)i; *E02B 1/00*(2006.01)i
FI:    A01M29/30; A01M29/10; E02B1/00 301Z

According to International Patent Classification (IPC) or to both national classification and IPC

| B. | FIELDS SEARCHED |
| --- | --- |

Minimum documentation searched (classification system followed by classification symbols)

A01M29/30; A01M29/10; E02B1/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | JP 2016-188570 A (THE CHUGOKU ELECTRIC POWER CO., INC.) 04 November 2016 (2016-11-04)<br>        paragraphs [0009]-[0029] | 1-5 |
| A | | 6-7 |
| A | WO 2014/027402 A1 (THE CHUGOKU ELECTRIC POWER CO., INC.) 20 February 2014 (2014-02-20)<br>        paragraphs [0006]-[0015] | 1-7 |
| A | JP 2010-207278 A (YAMAGUCHI UNIV) 24 September 2010 (2010-09-24)<br>        paragraphs [0036]-[0095] | 1-7 |
| A | JP 2010-187637 A (SESSILE RESEARCH CORP.) 02 September 2010 (2010-09-02)<br>        paragraphs [0012]-[0043] | 1-7 |

☐ Further documents are listed in the continuation of Box C.    ☑ See patent family annex.

| | |
| --- | --- |
| *    Special categories of cited documents: | |
| "A"  document defining the general state of the art which is not considered to be of particular relevance | "T"  later document published after the international filing date or priority date and not in conflict with the application but cited to understand the principle or theory underlying the invention |
| "E"  earlier application or patent but published on or after the international filing date | "X"  document of particular relevance; the claimed invention cannot be considered novel or cannot be considered to involve an inventive step when the document is taken alone |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search | Date of mailing of the international search report |
| --- | --- |
| **13 September 2023** | **14 November 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/032193**

| Patent document cited in search report | | | Publication date (day/month/year) | Patent family member(s) | Publication date (day/month/year) |
|---|---|---|---|---|---|
| JP | 2016-188570 | A | 04 November 2016 | (Family: none) | |
| WO | 2014/027402 | A1 | 20 February 2014 | US 2016/0143257 A1 paragraphs [0006]-[0015] EP 2885968 A1 CN 104735980 A KR 10-2017-0090521 A | |
| JP | 2010-207278 | A | 24 September 2010 | (Family: none) | |
| JP | 2010-187637 | A | 02 September 2010 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016188570 A